# EUROPEAN PATENT APPLICATION

(11) **EP 4 238 598 A1**
(43) Date of publication of application: **06.09.2023**
(21) Application number: 21894559.0
(22) Date of filing: 12.11.2021
(51) Int. Cl.: A61M 5/31, A61M 39/00, A61B 5/153

(54) **BUBBLE REMOVING CAP AND SYRINGE SET**

(30) Priority: 19.11.2020 JP 2020192596
(71) Applicant: TERUMO Kabushiki Kaisha, Tokyo 151-0072 (JP)
(72) Inventor: TAKAKI, Toshiaki, Nakakoma-gun, Yamanashi 409-3853 (JP); YASUDA, Yuzuri, Nakakoma-gun, Yamanashi 409-3853 (JP)
(74) Representative: Casalonga
(86) International application number: PCT/JP2021/041645
(87) International publication number: WO 2022/107685

(57) **Abstract**

An air bubble removal cap (12) includes: a tubular main body portion (30) having a hollow portion (38), connected to a collection tube (10) that accommodates a body fluid from a living body, therein; a connection portion (44) which is provided on a proximal side of the hollow portion (38) and to which the collection tube (10) is connected; an exhaust portion (46) that is provided on a distal side of the hollow portion (38) and discharges gas discharged from the collection tube (10); a filter (42) that is provided in the hollow portion (38) and divides the exhaust portion (46) and the connection portion (44); and a scattering prevention mechanism (48) that covers a proximal side of the filter (42) and prevents the body fluid from scattering to the filter (42).

## Description

### Technical Field

The present invention relates to an air bubble removal cap and a syringe set that remove air bubbles from a body fluid collected in a collection tube.

### Background Art

Conventionally, collection of a body fluid (for example, blood) has been performed for the purpose of confirming a condition of a patient or the like. The body fluid is collected in a collection tube (for example, a syringe), and air bubbles are removed from the body fluid collected in the collection tube in order to prevent an error in a measurement result and a malfunction of a measuring instrument.

The air bubbles are removed from the collection tube by placing a nozzle of the collection tube upward, tapping the collection tube with a finger or the like to collect the air bubbles in the vicinity of the nozzle, and then pushing a plunger (pusher) of the collection tube by applying gauze or the like to the nozzle. However, such an operation may expose a user to the body fluid of the patient, and there is a problem in safety.

Therefore, in order to more safely remove air bubbles from collection tubes, there is proposed a technique in which an air bubble removal cap is attached to a nozzle of a collection tube to remove air bubbles in the collection tube through the air bubble removal cap. An air bubble removal cap is described in ICU Medical Inc. (formerly, Smiths Medical Japan Ltd.), "Pro-Vent Plus", [online], searched on September 15, 2020, Internet, <URL: https://www.smiths-medical.com/-/media/M/Smiths-medical_com/Files/Import-Files/0201002_%E5%8B%95%E8%84%88%E8%A1%80%E6%8E%A1%E8%A1%80 %EF%BD%B7%EF%BD%AF%EF%BE%84%E3%81%94%E4%BD%BF%E7%94%A8%E6%9 6%B9%E6%B3%95_1707.pdf>.

An air bubble removal cap is also described in Radiometer K.K., "Arterial blood sampler safe PICO", [online], searched on September 15, 2020, Internet, <URL: https://www.info.pmda.go.jp/downfiles/md/PDF/840033/840033_ 21700BZY00533000_A_01_09.pdf>.

Further, JP 2017-506115 W describes a filter structure that can be attached to a medical connector or a nozzle of a syringe.

### Summary of Invention

The air bubble removal cap of "Pro-Vent Plus" described above enables exhaust of air bubbles while preventing leakage of blood with a filter that is clogged when coming into contact with the blood. However, the flow of the filter sometimes becomes worse before the exhaust of air bubbles in the air bubble removal cap of "Pro-Vent Plus" described above, and which makes it difficult to remove the air bubbles.

The air bubble removal cap of "Arterial blood sampler safe PICO" described above has a problem that a body fluid (blood) leaks from a distal end of the air bubble removal cap if a plunger is pushed too much at the time of discharging air bubbles.

Therefore, an object of the present invention is to provide an air bubble removal cap and a syringe set capable of safely and reliably removing air bubbles from a body fluid.

One aspect of the following disclosure is an air bubble removal cap including: a tubular main body portion having a hollow portion, connected to a collection tube that accommodates a body fluid from a living body, therein; a connection portion which is provided on a proximal side of the hollow portion and to which the collection tube is connected; an exhaust portion which is provided on a distal side of the hollow portion and discharges gas discharged from the collection tube; a filter which is disposed in the hollow portion and divides the exhaust portion and the connection portion; and a scattering prevention mechanism which covers a proximal side of the filter and prevents the body fluid from scattering to the filter.

Another aspect is a syringe set including: the air bubble removal cap according to the above aspect; and a collection tube that collects a body fluid.

According to the air bubble removal cap and the syringe set from the above aspects, the air bubbles can be safely and reliably removed from the body fluid collected in the collection tube.

### Brief Description of Drawings

Fig. 1 is a plan view of a syringe set according to a first embodiment.
Fig. 2A is a perspective view of an air bubble removal cap of Fig. 1, and Fig. 2B is a view of the air bubble removal cap of Fig. 1 as viewed from a proximal side.
Fig. 3 is a longitudinal cross-sectional view of the air bubble removal cap of Fig. 1.
Fig. 4 is a view of the air bubble removal cap of Fig. 1 in a state where a filter has been removed as viewed from a distal side.
Fig. 5 is a view illustrating an operation of collecting air bubbles at a distal end of the syringe attached with the air bubble removal cap of Fig. 1.
Fig. 6A is a schematic cross-sectional view (Part 1) illustrating a state of air bubble removal of the syringe using the air bubble removal cap of Fig. 1, and Fig. 6B is a schematic cross-sectional view (Part 2) illustrating a state of air bubble removal of the syringe using the air bubble removal cap of Fig. 1.
Fig. 7A is a schematic cross-sectional view (Part 3) illustrating a state of air bubble removal of the syringe using the air bubble removal cap of Fig. 1, and Fig. 7B is a schematic cross-sectional view (Part 4) illustrating a state of air bubble removal of the syringe using the air bubble removal cap of Fig. 1.
Fig. 8 is a longitudinal cross-sectional view of an air bubble removal cap according to a second embodiment.
Fig. 9A is a view of the air bubble removal cap of Fig. 8 as viewed from a proximal side, and Fig. 9B is a view of the air bubble removal cap of Fig. 8 in a state where a filter has been removed as viewed from a distal side.
Fig. 10A is a schematic cross-sectional view (Part 1) illustrating a state of air bubble removal of the syringe using the air bubble removal cap of Fig. 8, and Fig. 10B is a schematic cross-sectional view (Part 2) illustrating a state of air bubble removal of the syringe using the air bubble removal cap of Fig. 8.

### Description of Embodiments

Hereinafter, preferred embodiments of an air bubble removal cap and a syringe set will be described in detail with reference to the accompanying drawings. In the following description, in a state where an air bubble removal cap 12 is connected to a syringe 10 (collection tube), a direction which is closer to a user will be referred to as a "proximal direction" or a "proximal side", and a direction in which an exhaust portion 46 of the air bubble removal cap 12 is disposed will be referred to as a "distal direction" or a "distal side".

### (First Embodiment)

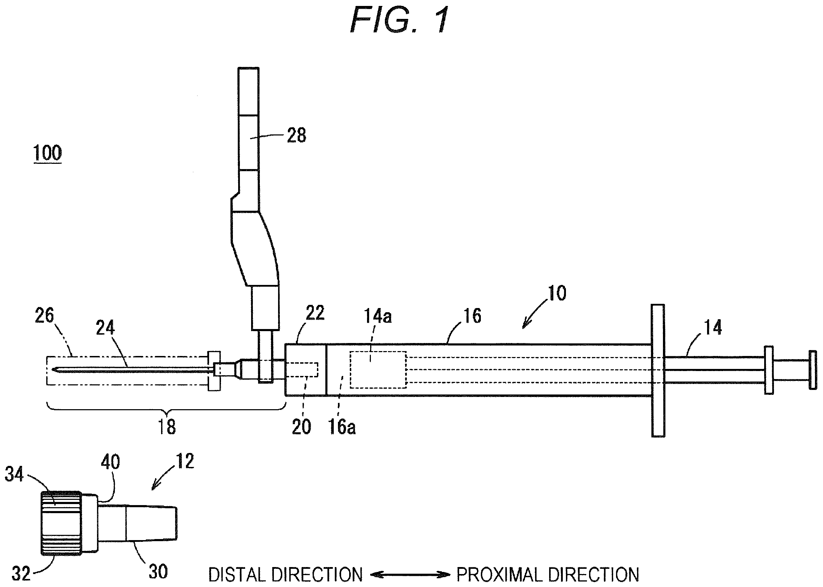

As illustrated in Fig. 1, a syringe set 100 according to the present embodiment includes the syringe 10 (collection tube) that collects a body fluid of a living body and the air bubble removal cap 12. The syringe 10 is, for example, a blood sampling syringe that collects blood. The syringe 10 includes a plunger 14, a syringe body 16 into which the plunger 14 is inserted, and a needle module 18.

The syringe 10 can perform blood sampling by arterial pressure and suction of blood by a suction operation of drawing the plunger 14. A gasket 14a of the plunger 14 is provided with a vent filter (not illustrated) that enables discharge of air and is clogged when coming into contact with the blood. The plunger 14 is formed in a hollow shape, and can discharge air (air bubbles) expelled from the syringe body 16 by arterial pressure (through the vent filter).

The syringe body 16 is formed in a hollow cylindrical shape and has an empty chamber 16a formed inside. The plunger 14 is inserted into the empty chamber 16a to be axially slidable. A drug that prevents blood coagulation, such as heparin, is sealed in the empty chamber 16a of the syringe body 16. A nozzle 20 (tip) having a small diameter is formed at a distal end of the syringe body 16. The diameter of the nozzle 20 is reduced in a tapered shape such that the outer diameter gradually decreases toward the distal end. An adapter 22 in which a female screw is formed is provided outside the nozzle 20.

The needle module 18 is attached to the nozzle 20. The needle module 18 includes a needle member 24 that communicates with the nozzle 20, a protective cap 26 that covers the needle member 24 and is removed immediately before use, and a protector 28 that prevents erroneous puncture after use. The needle module 18 is screwed into the adapter 22 and is removed from the syringe body 16 after the blood sampling is completed.

The air bubble removal cap 12 is attached to the nozzle 20 of the syringe body 16 from which the needle module 18 is removed after completion of the blood sampling, and is used to remove air bubbles of the blood in the syringe 10.

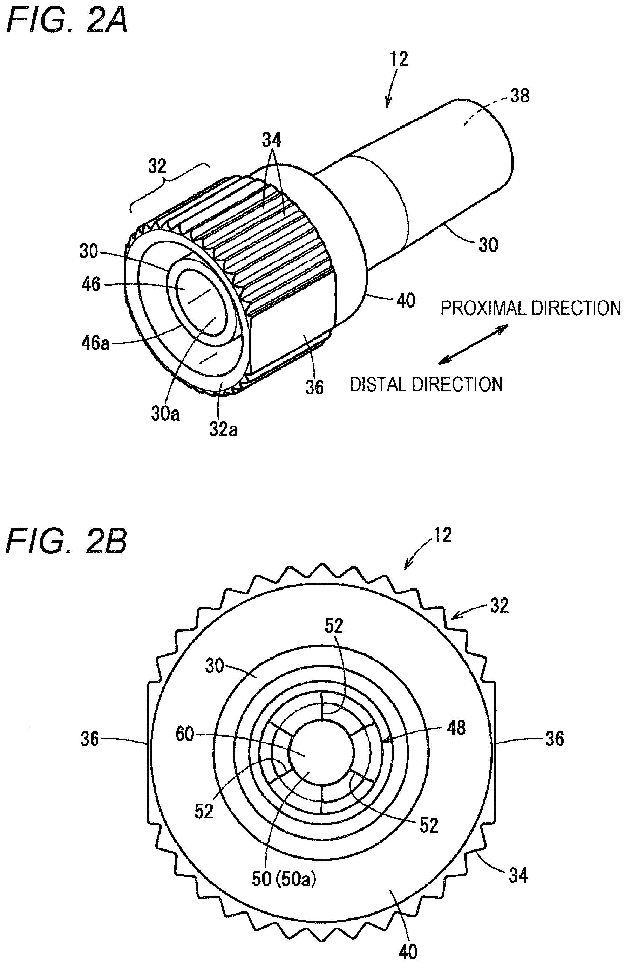

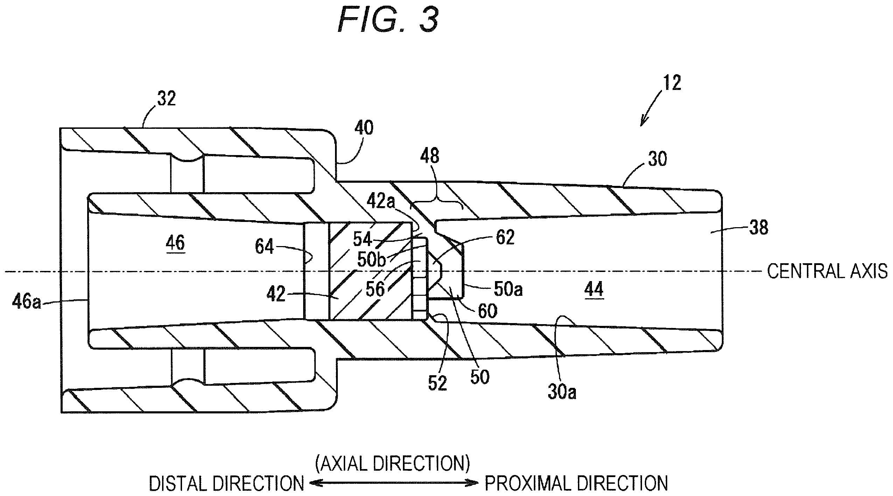

As illustrated in Fig. 2A, the air bubble removal cap 12 has a main body portion 30 having a cylindrical shape and a grip portion 32 provided on the distal side of the main body portion 30. A hollow portion 38 having a circular cross section is formed inside the main body portion 30. As illustrated in Fig. 3, the hollow portion 38 penetrates and extends along a central axis from the distal side to the proximal side. Note that the central axis of the air bubble removal cap 12 is simply referred to as the "central axis" in the present specification and the drawings. As illustrated in Fig. 2A, the grip portion 32 is formed in a cylindrical shape having an inner diameter larger than an outer diameter of the main body portion 30, and surrounds the outer side of the main body portion 30 on the distal side. Although not particularly limited, the main body portion 30 and the grip portion 32 are made of a transparent or translucent resin material (for example, a polypropylene resin, a PET resin, or the like) which enables visual recognition of the inside.

As illustrated in Figs. 2A and 2B, a distal portion 32a of the grip portion 32 protrudes more toward the distal side than a distal end of the main body portion 30, and is formed so as to prevent the main body portion 30 from being in contact with a finger or the like. The grip portion 32 is joined to the vicinity of the axial center of the main body portion 30 via a shoulder portion 40 provided at a proximal end. The grip portion 32 includes, at an outer peripheral portion, a knurled portion 34 in which a plurality of grooves extending in the axial direction are formed in order to prevent a sip when being gripped. Further, a flat cutout portion 36, that prevents rolling of the air bubble removal cap 12, is formed in a part of the outer peripheral portion of the grip portion 32 in the circumferential direction.

As illustrated in Fig. 3, a filter 42 is provided in the vicinity of the axial center of the hollow portion 38 of the main body portion 30. The filter 42 is in close contact with an inner peripheral surface 30a of the hollow portion 38, and partitions the hollow portion 38 into a connection portion 44 on the proximal side and the exhaust portion 46 on the distal side. The filter 42 is made of a porous material obtained by sintering a hydrophilic resin (for example, a starch-sodium acrylate graft copolymer), a water-absorbent polymer material, and a binder resin (for example, polyethylene). In a dry state, pores of the filter 42 communicate with each other so that gas (air) can pass from the proximal side to the distal side. When the filter 42 comes into contact with the body fluid, the water-absorbent polymer material swells to close the pores, thereby clogging the passage of gas and liquid.

The connection portion 44 on the proximal side of the filter 42 is surrounded by a tapered smooth inner peripheral surface 30a whose inner diameter gradually increases toward the proximal side. The tapered nozzle 20 of the syringe 10 is liquid-tightly and airtightly connected to the connection portion 44. A scattering prevention mechanism 48 formed to protrude from the inner peripheral surface 30a of the main body portion 30 toward the hollow portion 38 is formed in the vicinity of a distal end of the connection portion 44. The scattering prevention mechanism 48 includes a barrier portion 50 formed in a wall shape, a narrow flow path 52 hollowed out to axially penetrate through a part of the barrier portion 50, and a protruding portion 54 forming a gap 56 between the barrier portion 50 and the filter 42.

On a proximal end surface 50a of the barrier portion 50, a protrusion 60 in which the vicinity of the central axis protrudes toward the proximal side is formed. Further, on a distal end surface 50b of the barrier portion 50, a recess 62 in which the vicinity of the central axis is recessed toward the proximal side is provided on the opposite side of the protrusion 60. A peripheral edge of the barrier portion 50 is integrally connected to the main body portion 30.

As illustrated in Fig. 2B, the narrow flow path 52 is provided in the vicinity of the peripheral edge of the barrier portion 50. Three narrow flow paths 52 of the present embodiment are provided at an angle of 120° in the circumferential direction. The number of the narrow flow paths 52 is not limited to three, and may be one or two. Further, four or more narrow flow paths 52 may be provided.

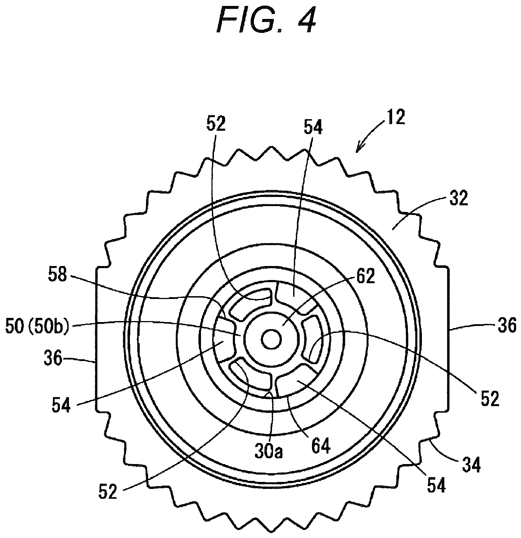

As illustrated in Fig. 3, the protruding portion 54 is formed to be adjacent to the distal side of barrier portion 50 and integrally connected to barrier portion 50. As illustrated in Fig. 4, the protruding portion 54 is formed to protrude short from the inner peripheral surface 30a of the main body portion 30 toward the center side. Three protruding portions 54 are provided separately in the circumferential direction, and are disposed at parts avoiding the narrow flow path 52. A flow channel 58 is formed between the adjacent protruding portions 54. The flow channel 58 communicates with a proximal end of the narrow flow path 52. As illustrated in Fig. 3, a distal end of the protruding portion 54 comes into contact with a proximal end surface 42a of the filter 42 to form the gap 56 between the filter 42 and the barrier portion 50. Note that the protruding portion 54 is not limited to one integrally formed with barrier portion 50 as illustrated in the drawings, and may be formed by joining another member to the barrier portion 50.

As illustrated in Figs. 2A and 3, the exhaust portion 46 is provided with an opening 46a on the distal side, and exhausts the gas that has passed through the filter 42 through the opening 46a. Further, the exhaust portion 46 is provided with an undercut portion 64 formed by a step protruding inward from the inner peripheral surface 30a. The undercut portion 64 prevents the filter 42 from dropping out. As illustrated in Fig. 4, the undercut portion 64 is formed in a ring shape over the entire region in the circumferential direction, but is not limited thereto, and may be formed separately in the circumferential direction. The undercut portion 64 is formed to have such a protruding height (in the radial direction) as to prevent the filter 42 from getting over the undercut portion 64 even when the filter 42 absorbs the body fluid and expands. Further, the undercut portion 64 is provided at a position spaced apart from the filter 42 in the dry state toward the distal side by a predetermined distance in consideration of the expansion of the filter 42.

The air bubble removal cap 12 and the syringe set 100 of the present embodiment are configured as described above, and the operational effect thereof will be described together with a way to use hereinafter.

The syringe set 100 is provided with the syringe 10 for blood sampling and the air bubble removal cap 12 illustrated in Fig. 1 as a set. A user, such as a doctor or a nurse, removes the protective cap 26 of the syringe 10 and punctures a blood vessel, such as an artery of a patient, with the needle member 24 to perform the blood sampling. Note that the syringe 10 may be connected to a connection port of a blood sampling line to perform the blood sampling. In this case, the user can perform the blood sampling by removing the needle module 18 from the syringe 10 and connecting the nozzle 20 of the syringe 10 to the connection port of the blood sampling line.

The blood collected in the syringe 10 is used, for example, to examine an oxygen concentration or the like in the blood of the patient. If air bubbles remain inside the syringe 10, the oxygen concentration is likely to be excessively detected to cause an error in a measurement result. Therefore, it is desirable to perform an operation of quickly removing the air bubbles inside the syringe 10 after the blood sampling is completed.

In the present embodiment, the user attaches the air bubble removal cap 12 to the nozzle 20 of the syringe 10 to perform removal of the air bubbles in order to safely remove the air bubbles. Prior to the attachment of the air bubble removal cap 12, the user attaches the protector 28 to the needle member 24 of Fig. 1 and removes the needle module 18 from the syringe 10. Then, the user pushes the nozzle 20 of the syringe 10 into the hollow portion 38 on the proximal side of the air bubble removal cap 12 to connect the syringe 10 and the air bubble removal cap 12.

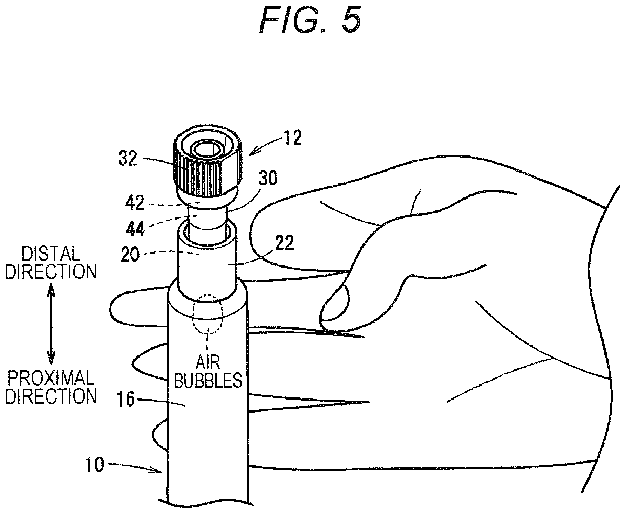

Next, as illustrated in Fig. 5, the user grips the syringe 10 in an upright state with one hand such that the air bubble removal cap 12 is on the upper side, and applies vibration by, for example, tapping the syringe 10 with a finger of the other hand to collect the air bubbles inside the syringe 10 to the vicinity of the nozzle 20.

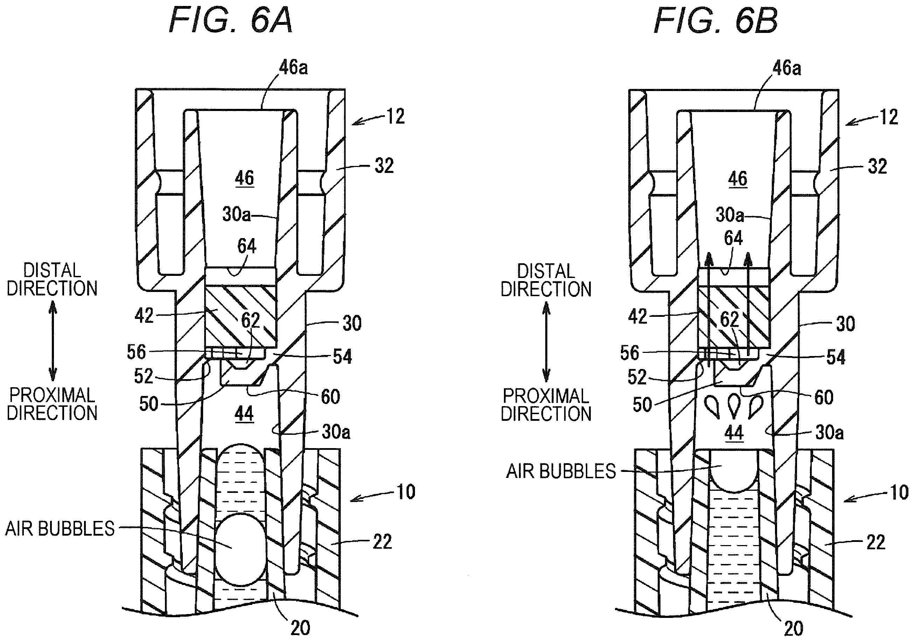

Next, the user pushes the plunger 14 to discharge the air bubbles collected in the vicinity of the nozzle 20 of the syringe 10 from the syringe 10. As illustrated in Fig. 6A, when the air bubbles are discharged from the syringe 10, the air bubbles are discharged together with the blood in the syringe 10 and the nozzle 20. If the blood discharged from the nozzle 20 scatters and comes into contact with the filter 42 before completion of the discharge of the air bubbles, the filter 42 is clogged.

On the other hand, the air bubble removal cap 12 of the present embodiment is provided with the barrier portion 50 constituting a part of the scattering prevention mechanism 48 on the proximal side of the filter 42 as illustrated in Fig. 6B, and most of the filter 42 on the proximal side is covered with the barrier portion 50. The barrier portion 50 prevents droplets of the blood from moving to the distal side so that the filter 42 can be prevented from being clogged. The air bubbles pass through the narrow flow path 52 penetrating through the barrier portion 50 and are discharged from the exhaust portion 46 via the filter 42.

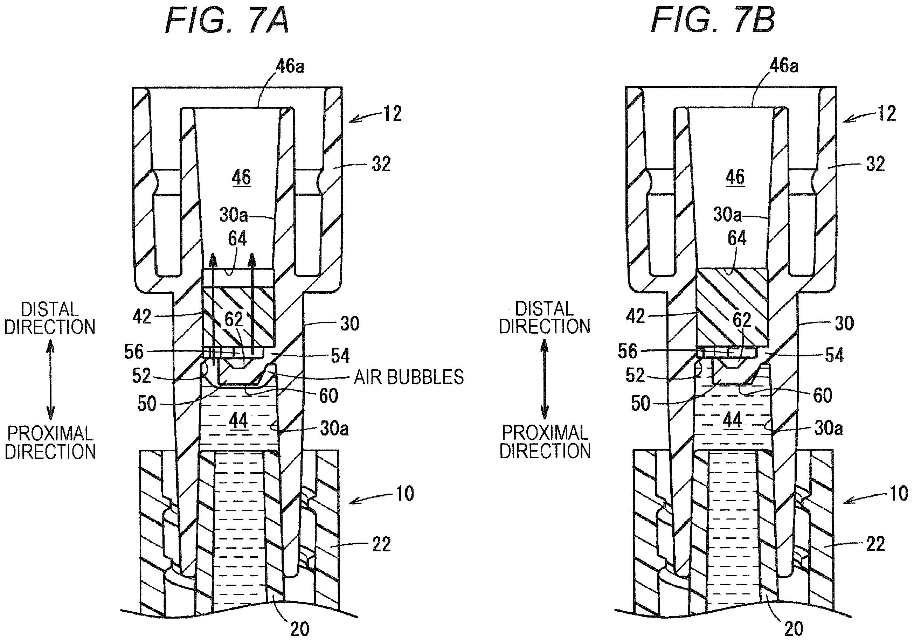

When the plunger 14 is further pushed from the state of Fig. 6B as illustrated in Fig. 7A, the blood flows out from the nozzle 20, the liquid level of the blood rises in the connection portion 44, and gas in the connection portion 44 is discharged. In the connection portion 44, the liquid level of the blood is higher in the vicinity of a boundary with an inner peripheral wall of the connection portion 44 due to surface tension, and is lower in the vicinity of the central axis. In the present embodiment, the protrusion 60 protruding in accordance with the liquid level of the blood is formed on the proximal side of the proximal end surface 50a of the barrier portion 50. As a result, air in the connection portion 44 can be discharged without leaving the air in the connection portion 44.

Further, the blood scattered in the process of discharging the air illustrated in Figs. 6B and 7A passes through the narrow flow path 52 in some cases. Even if such an event occurs, the blood can be prevented from coming into contact with the filter 42 before completion of the discharge of the air since the gap 56 is formed between the filter 42 and the narrow flow path 52 (barrier portion 50) due to the protruding portion 54. The blood that has passed through the narrow flow path 52 is stored in the recess 62 provided in the distal end surface 50b of the barrier portion 50, so that the contact of the blood with the filter 42 can be more effectively prevented.

When the user further pushes the plunger 14, the air on the connection portion 44 side is completely discharged, and the blood comes into contact with the filter 42 as illustrated in Fig. 7B. As a result, the filter 42 absorbs moisture in the blood and swells, thereby liquid-tightly and airtightly clogging the connection portion 44 and the exhaust portion 46. As a result, leakage of blood to the exhaust portion 46 side is prevented, the user can remove the air bubbles without being exposed to the blood. Note that the filter 42 in the swollen state is prevented from moving to the distal side by the undercut portion 64 on the distal side. Therefore, it is difficult to push the plunger 14 any more if the filter 42 is clogged. The user can confirm the completion of discharge of the air bubbles based on discoloration of the filter 42 caused by the blood and the stop of the plunger 14 caused by the clogging of the filter 42.

The syringe 10 is carried with the air bubble removal cap 12 attached while preventing the flow of the air bubbles into the syringe 10. The air bubble removal cap 12 is removed from the syringe 10 immediately before the syringe 10 is set in devices such as an examination device. Thereafter, the blood of the syringe 10 is used for examination of the oxygen concentration or the like.

The air bubble removal cap 12 and the syringe set 100 of the present embodiment have the following effects.

The air bubble removal cap 12 of the present embodiment includes: the tubular main body portion 30 having the hollow portion 38, connected to the syringe 10 (collection tube) that accommodates blood (body fluid) from a living body, therein; the connection portion 44 which is provided on the proximal side of the hollow portion 38 and to which the syringe 10 is connected; the exhaust portion 46 that is provided on the distal side of the hollow portion 38 and discharges gas discharged from the syringe 10; the filter 42 that is disposed in the hollow portion 38 and divides the exhaust portion 46 and the connection portion 44; and the scattering prevention mechanism 48 that covers the proximal side of the filter 42 and prevents the body fluid from scattering to the filter 42.

The above-described air bubble removal cap 12 prevents adhesion of the blood scattered from the nozzle 20 by the scattering prevention mechanism 48. As a result, it is possible to prevent an event in which the filter 42 is clogged before the air bubbles are discharged, and to reliably discharge the air bubbles from the syringe 10.

In the above-described air bubble removal cap 12, the syringe 10 (collection tube) is inserted into the hollow portion 38 from a side of the filter 42 closer to the scattering prevention mechanism 48. According to this configuration, the scattering prevention mechanism 48 is disposed between the filter 42 and the syringe 10, and thus, it is possible to prevent the filter 42 from being clogged by the body fluid scattered from the syringe 10.

In the above-described air bubble removal cap 12, the scattering prevention mechanism 48 may have the narrow flow path 52 having a smaller diameter than the hollow portion 38. According to this configuration, a width through which the scattered blood can pass is narrowed, and thus, the adhesion of the blood to the filter 42 can be suppressed.

In the air bubble removal cap 12, the scattering prevention mechanism 48 may have the wall-shaped barrier portion 50 that separates the connection portion 44 and the filter 42, and the narrow flow path 52 may be formed penetrating through the barrier portion 50. According to this configuration, the filter 42 is protected from the scattering blood by the barrier portion 50, so that the air bubbles can be reliably discharged from the syringe 10.

In the above-described air bubble removal cap 12, when the hollow portion 38 is viewed from the axial direction, the barrier portion 50 has a larger area than the narrow flow path 52. According to this configuration, the barrier portion 50 can protect the filter 42 from the scattering blood.

In the above-described air bubble removal cap 12, the scattering prevention mechanism 48 may have the protruding portion 54 that is provided on the proximal side of the filter 42 and forms the gap 56 between the filter 42 and the narrow flow path 52. According to this configuration, even if the blood passes through the narrow flow path 52, the filter 42 can be immediately prevented from being clogged.

In the above-described air bubble removal cap 12, the protruding portion 54 is formed protruding from the inner peripheral surface 30a of the main body portion 30 toward the central axis, and the barrier portion 50 further protrudes toward the central axis with respect to the protruding portion 54. According to this configuration, the barrier portion 50 can protect the filter 42 from the scattering blood.

In the above-described air bubble removal cap 12, the protruding portion 54 may be provided on the distal side of the scattering prevention mechanism 48. That is, since the protruding portion 54 is provided on a side of the scattering prevention mechanism 48 closer to the filter 42, the filter 42 is less likely to come into contact with the blood, and the filter 42 can be prevented from being clogged until the removal of air bubbles is completed.

In the above-described air bubble removal cap 12, the protruding portion 54 may be formed so as to protrude from the inner peripheral surface 30a of the main body portion 30 to the central axis. According to this configuration, the protruding portion 54 can be integrally formed with the air bubble removal cap 12 by injection molding.

In the above-described air bubble removal cap 12, the protruding portion 54 may be in contact with the filter 42. With this configuration, a proximal end of the filter 42 and the narrow flow path 52 of the scattering prevention mechanism 48 can be spaced apart from each other.

In the above-described air bubble removal cap 12, the protrusion 60 in which the vicinity of the central axis protrudes to the proximal side from the outer peripheral portion may be formed on the proximal side of the barrier portion 50. Since the protrusion 60 protrudes along the liquid level of the blood, the air can be discharged without leaving the air bubbles in the connection portion 44.

In the above-described air bubble removal cap 12, the scattering prevention mechanism 48 may have the recess 62 recessed toward the proximal side so as to be spaced apart from the filter 42 on the distal side of the barrier portion 50. Since the blood that has passed through the narrow flow path 52 is accommodated in the recess 62, it is possible to prevent the filter 42 from being clogged.

In the above-described air bubble removal cap 12, an end of the recess 62 on the proximal side may be located on the proximal side with respect to an end of the narrow flow path 52 on the distal side. That is, the recess 62 and the narrow flow path 52 may be disposed at different positions. According to this configuration, the air can pass through the narrow flow path 52 without being hindered by the blood stored in the recess 62, and further scattering of the blood can be suppressed in the recess 62.

In the above-described air bubble removal cap 12, the scattering prevention mechanism 48 includes: the wall-shaped barrier portion 50 that separates the connection portion 44 and the filter 42; the plurality of narrow flow paths 52 that are formed in the outer peripheral portion of the barrier portion 50 and penetrate through the barrier portion 50 in the axial direction; and the protruding portion 54 that protrudes toward the central axis from the inner peripheral surface 30a of the main body portion 30 provided on the distal side of the barrier portion 50 and at a position avoiding the circumferential positions of the narrow flow paths 52 to form the gap 56 between each of the narrow flow paths 52 and the filter 42, and the recess 62 recessed toward the proximal side may be formed in the vicinity of the central axis of the end surface on the distal side of the barrier portion 50.

In the above-described air bubble removal cap 12, the protruding portion 54 may be provided on the distal side with respect to the recess 62. According to this configuration, since the gap 56 is formed between the recess 62 and the filter 42, it is possible to prevent the filter 42 from being clogged in the middle of exhaust.

In the above-described air bubble removal cap 12, the main body portion 30 may have a filter fixing portion (for example, the undercut portion 64), which prevents movement of the filter 42 to the distal side, on the distal side of the filter 42. In this case, the filter fixing portion may have the undercut portion 64 formed of a step protruding inward from the inner peripheral surface 30a of the main body portion 30. According to this configuration, even if the plunger 14 is pushed, it is possible to prevent the filter 42 from dropping out and to prevent leakage of the blood at the time of removal of the air bubbles.

In the above-described air bubble removal cap 12, the connection portion 44 may have a lock mechanism (for example, a screw structure capable of engaging with a female screw on the outer side of the nozzle 20 of the syringe 10 or a luer taper) capable of fixing the syringe 10. According to this configuration, the syringe 10 can be reliably fixed, and the air bubble removal cap 12 can be prevented from dropping out.

In the above-described air bubble removal cap 12, the lock mechanism is formed by being reduced in diameter in a tapered shape toward a distal end. As a result, the air bubble removal cap 12 having excellent versatility and connectable to various types of the syringes 10 can be realized.

In the above-described air bubble removal cap 12, the grip portion 32 protruding outward from the main body portion 30 may be formed. According to this configuration, since the grip portion 32 having a thickness that can be easily gripped is provided, handleability of the air bubble removal cap 12 is improved. In the air bubble removal cap 12, a plurality of the knurled portions 34 spaced apart in the circumferential direction may be provided at the outer peripheral portion of the grip portion 32. According to this configuration, it is possible to prevent slippage when the grip portion 32 is gripped, and the handleability of the grip portion 32 is further improved. Further, in the above-described air bubble removal cap 12, the cutout portion 36 obtained by cutting out a part of the grip portion 32 in the circumferential direction to be flat may be provided.

The syringe set 100 of the present embodiment includes the above-described air bubble removal cap 12 and the syringe 10 that collects the body fluid. According to the syringe set 100, the user can safely and reliably remove the air bubbles of the syringe 10.

### (Second Embodiment)

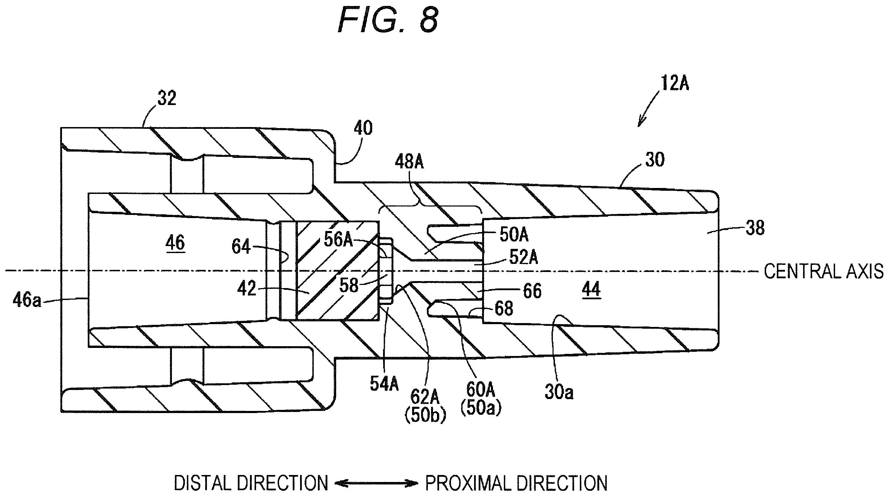

As illustrated in Fig. 8, an air bubble removal cap 12A of the present embodiment is different from the air bubble removal cap 12 described with reference to Figs. 1 to 4 in terms of a structure of a scattering prevention mechanism 48A. Note that an appearance of the air bubble removal cap 12A is similar to that illustrated in Fig. 2A. In the air bubble removal cap 12A, configurations similar to those of the air bubble removal cap 12 will be denoted by the same reference signs, and the detailed description thereof will be omitted.

As illustrated in Fig. 8, the hollow portion 38 extending in the axial direction is formed inside the main body portion 30 of the air bubble removal cap 12A. The filter 42 is provided in the vicinity of the axial center of the hollow portion 38, and the scattering prevention mechanism 48A of the present embodiment is formed on the proximal side of the filter 42.

The scattering prevention mechanism 48A includes a barrier portion 50A formed in a wall shape. The barrier portion 50A is formed in a funnel shape such that the vicinity of the central axis protrudes toward the proximal side. An end surface of the barrier portion 50A on the distal side is formed as a recess 62A recessed toward the proximal side in a conical shape. Further, a protrusion 60A protruding toward the proximal side in a conical shape is formed on an end surface of the barrier portion 50A on the proximal side.

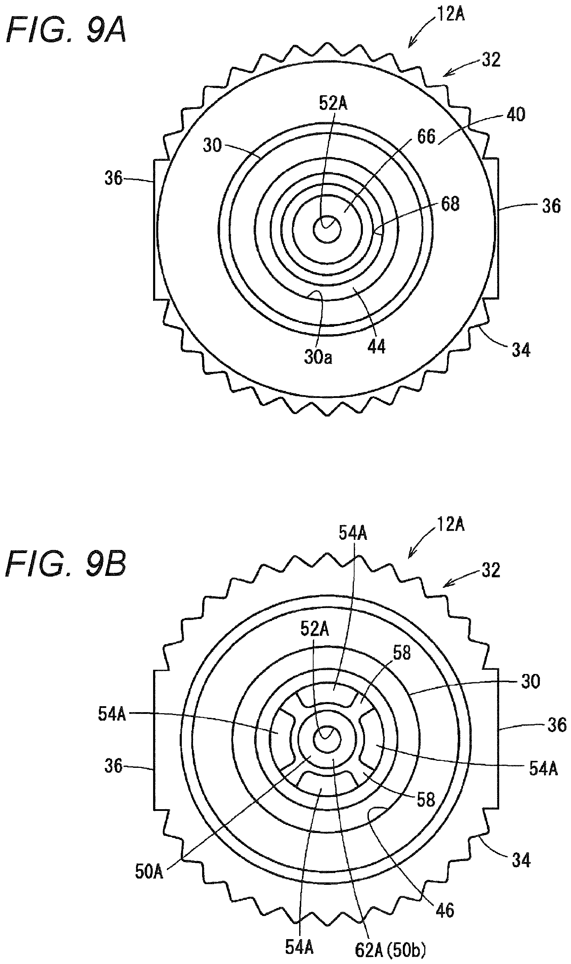

A tubular portion 66 extending in the axial direction toward the proximal side is provided from the vicinity of the central axis on the proximal side of the barrier portion 50A. An outer diameter of the tubular portion 66 is formed to be smaller than an inner diameter of the connection portion 44, and an annular groove portion 68 is provided between the tubular portion 66 and the inner peripheral surface 30a of the main body portion 30 as illustrated in Fig. 9A. Further, a narrow flow path 52A is formed in a central portion of the tubular portion 66. The narrow flow path 52A is formed as a hollow hole having a circular cross section. Note that the tubular portion 66 is not limited to one formed integrally with the barrier portion 50A as illustrated in the drawing, and may be formed by joining another member to the barrier portion 50A.

As illustrated in Fig. 8, the narrow flow path 52A extends along the central axis and penetrates through the tubular portion 66 and the barrier portion 50A. A distal end of the narrow flow path 52A is connected to the proximal side of the recess 62A of the barrier portion 50A, and the narrow flow path 52A is located on the proximal side with respect to an end of the recess 62A on the distal side. The connection portion 44 and the exhaust portion 46 communicate with each other through the narrow flow path 52A.

A protruding portion 54A forming a gap 56A is provided between the barrier portion 50A and the filter 42. As illustrated in Fig. 9B, a plurality of the protruding portions 54A are provided apart from each other in the circumferential direction. The flow channel 58 is formed between the protruding portions 54A spaced apart in the circumferential direction. Note that the protruding portions 54A of the present embodiment may be formed in a circular ring shape integrally connected in the circumferential direction.

As illustrated in Fig. 8, the filter 42 is disposed so as to be in contact with the protruding portion 54A, and the gap 56A having a predetermined capacity is formed between the filter 42 and the recess 62A.

The air bubble removal cap 12A of the present embodiment is configured as described above, and the operational effect thereof will be described hereinafter.

As described with reference to Figs. 5 to 7B, the air bubble removal cap 12A of the present embodiment is connected to the nozzle 20 of the syringe 10 and used for removing air bubbles of blood collected in the syringe 10.

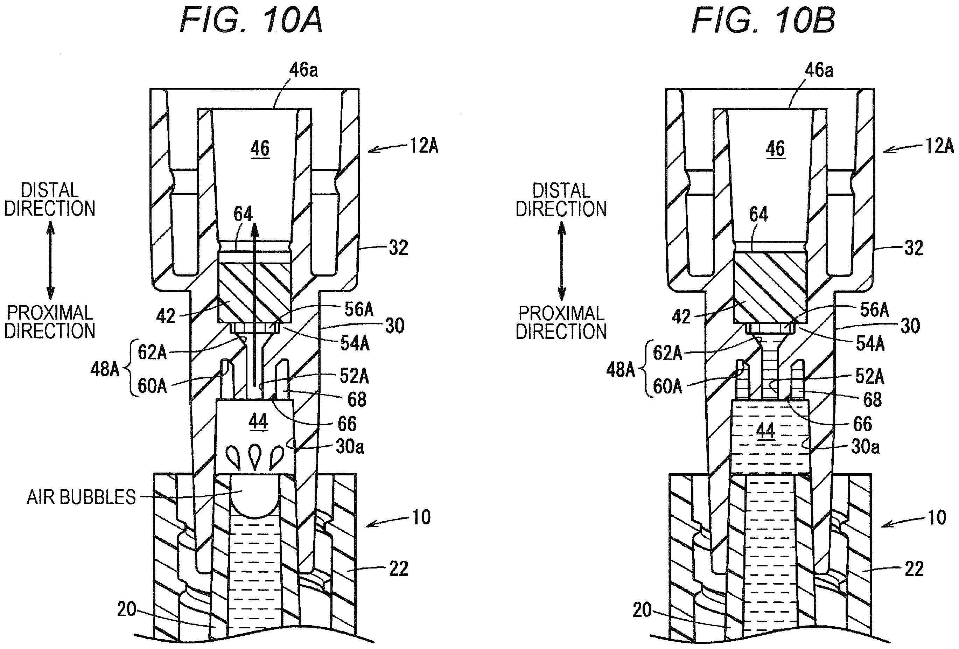

As illustrated in Fig. 10A, in the air bubble removal cap 12A of the present embodiment, the narrow flow path 52A extends long in the axial direction along the central axis of the tubular portion 66, and thus, the blood scattered from the nozzle 20 is less likely to directly pass therethrough. As a result, the air bubble removal cap 12A can more effectively prevent the filter 42 from being clogged before the air bubbles removal.

As illustrated in Fig. 10B, when the plunger 14 of the syringe 10 is further pushed, air and the air bubbles in a part communicating with the filter 42 are discharged from the exhaust portion 46 through the filter 42. Thereafter, the blood comes into contact with the filter 42, so that the filter 42 is clogged, and the air bubble removal is completed.

The air bubble removal cap 12A of the present embodiment has the following effects.

In the air bubble removal cap 12A of the present embodiment, an end of the recess 62A on the proximal side is located on the proximal side with respect to the end of the narrow flow path 52A on the distal side. This configuration also provides the air bubble removal cap 12A that can safely and reliably remove the air bubbles.

In the above-described air bubble removal cap 12A, the end of the recess 62A on the proximal side communicates with the narrow flow path 52A. According to this configuration, the recess 62A is disposed between the narrow flow path 52A and the filter 42, and thus, it is easy to secure a distance between the narrow flow path 52A and the filter 42, and it is possible to prevent the filter 42 from being clogged before the air bubbles are removed.

The above-described air bubble removal cap 12A may include the tubular portion 66 that extends in the axial direction from the vicinity of the central axis of the barrier portion 50A to the proximal side and has an outer peripheral portion spaced apart from the inner peripheral surface 30a of the main body portion 30, and the narrow flow path 52A may be formed so as to penetrate through the central axis of the tubular portion 66 in the axial direction. According to this configuration, it is possible to prevent the occurrence of a sink mark when the air bubble removal cap 12A is formed by resin molding by being formed using the thin tubular portion 66, and it is possible to enhance stability of a shape. Further, since the narrow flow path 52A is formed to be long in the axial direction, the scattering blood is less likely to pass through the narrow flow path 52A.

Although the present invention has been described with the preferred embodiments as above, it is obvious that the present invention is not limited to the above-described embodiments, and various modifications can be made within a scope not departing from a gist of the present invention.

## Claims

1. An air bubble removal cap comprising:
a main body portion which is formed in a tubular shape and has a hollow portion inside, the hollow portion being connected to a collection tube that accommodates a body fluid from a living body;
a connection portion which is provided on a proximal side of the hollow portion and to which the collection tube is connected;
an exhaust portion which is provided on a distal side of the hollow portion and discharges gas discharged from the collection tube;
a filter which is disposed in the hollow portion and divides the exhaust portion and the connection portion; and
a scattering prevention mechanism which covers a proximal side of the filter and prevents the body fluid from scattering to the filter.

2. The air bubble removal cap according to claim 1, wherein the collection tube is inserted into the hollow portion from a side of the filter closer to the scattering prevention mechanism.

3. The air bubble removal cap according to claim 1 or 2, wherein the scattering prevention mechanism includes a narrow flow path having a smaller diameter than the hollow portion.

4. The air bubble removal cap according to claim 3, wherein the scattering prevention mechanism includes a barrier portion which is formed in a wall shape and separates the connection portion and the filter, and the narrow flow path is formed penetrating through the barrier portion.

5. The air bubble removal cap according to claim 4, wherein an area of the barrier portion is larger than an area of the narrow flow path when the hollow portion is viewed from an axial direction.

6. The air bubble removal cap according to claim 4 or 5, wherein the scattering prevention mechanism has a protruding portion that is provided on the proximal side of the filter and forms a gap between the filter and the narrow flow path.

7. The air bubble removal cap according to claim 6, wherein the protruding portion is formed protruding from an inner peripheral surface of the main body portion toward a central axis, and the barrier portion further protrudes toward the central axis with respect to the protruding portion.

8. The air bubble removal cap according to claim 6 or 7, wherein the protruding portion is provided on a distal side of the scattering prevention mechanism.

9. The air bubble removal cap according to any one of claims 6 to 8, wherein the protruding portion is in contact with the filter.

10. The air bubble removal cap according to claim 4, wherein the barrier portion has a protrusion on a proximal side, the protrusion being formed to have a vicinity of a central axis protruding to the proximal side with respect to an outer peripheral portion.

11. The air bubble removal cap according to claim 4, wherein the scattering prevention mechanism has a recess on a distal side of the barrier portion, the recess being recessed toward a proximal side to be spaced apart from the filter.

12. The air bubble removal cap according to claim 11, wherein an end of the recess on the proximal side is located on the proximal side with respect to an end of the narrow flow path on a distal side.

13. The air bubble removal cap according to claim 11, wherein an end of the recess on the proximal side communicates with the narrow flow path.

14. The air bubble removal cap according to claim 1, wherein
the scattering prevention mechanism includes: a wall-shaped barrier portion that separates the connection portion and the filter; a plurality of narrow flow paths that are formed in an outer peripheral portion of the barrier portion and penetrate through the barrier portion in an axial direction; and a protruding portion that is provided on a distal side of the barrier portion and protrudes toward a central axis from an inner peripheral surface of the main body portion at a position avoiding circumferential positions of the narrow flow paths to form a gap between each of the narrow flow paths and the filter, and
a recess recessed toward a proximal side is formed in a vicinity of the central axis of an end surface on the distal side of the barrier portion.

15. The air bubble removal cap according to any one of claims 1 to 14, wherein the main body portion includes a filter fixing portion on a distal side of the filter to prevent the filter from moving to the distal side.

16. The air bubble removal cap according to claim 15, wherein the filter fixing portion includes an undercut portion formed of a step protruding inward from the inner peripheral surface of the main body portion.

17. The air bubble removal cap according to any one of claims 1 to 16, wherein the connection portion has a lock mechanism capable of fixing the collection tube.

18. The air bubble removal cap according to claim 17, wherein the lock mechanism is formed by being reduced in diameter in a tapered shape toward a distal end.

19. A syringe set comprising: the air bubble removal cap according to any one of claims 1 to 18; and a collection tube that collects a body fluid.
